# EUROPÄISCHE PATENTSCHRIFT

(11) **EP 2 603 798 B1**
(45) Veröffentlichungstag und Bekanntmachung des Hinweises auf die Patenterteilung: **20.01.2016**
(21) Anmeldenummer: 11751789.6
(22) Anmeldetag: 16.08.2011
(51) Int. Cl.: G01N 33/68, G01N 33/543

(54) **KIT UMFASSEND EINE TESTVORRICHTUNG ZUM FESTSTELLEN DER SCHWANGERSCHAFTSWOCHE**
KIT COMPRISING A TESTING DEVICE FOR DETERMINING THE WEEK OF PREGNANCY
KIT COMPRENANT UN DISPOSITIF DE TEST POUR DÉTERMINER LA SEMAINE DE GROSSESSE

(30) Priorität: 13.08.2010 AT 13602010
(43) Veröffentlichungstag der Anmeldung: 19.06.2013
(73) Patentinhaber: Scheuringer, Kim, 2513 Möllersdorf (AT)
(72) Erfinder: Scheuringer, Kim, 2513 Möllersdorf (AT)
(74) Vertreter: Sonn & Partner Patentanwälte
(86) Internationale Anmeldenummer: PCT/AT2011/000345
(87) Internationale Veröffentlichungsnummer: WO 2012/019212

(56) Entgegenhaltungen:
- EP-A1- 0 225 054
- WO-A1-2006/100415
- GB-A- 2 460 660
- US-A1- 2010 129 935
- Care diagnostica: "Viola Schwangerschaftsfrühtest Varianten der Testdurchführung", F.Uhlmann-Eyraud S.A Product Catalog , August 2005 (2005-08), Seiten 1-2, XP002662959, Gefunden im Internet: URL:http://www.uhlmann.ch/index.php?page=s hop.getfile&file_id=48&product_id=133&opti on=com_virtuemart&Itemid=2 [gefunden am 2011-11-04]
- Dipl. MTA Zeilinger M: "HCG Referenzberiche ("Normalbereiche") Plasma/Serum", MED4YOU LABORBEFUNDE , 14. Dezember 2004 (2004-12-14), Seiten 1-4, XP002662960, Gefunden im Internet: URL:http://www.med4you.at/laborbefunde/ref erenzwerte/referenzbereiche_hcg.htm [gefunden am 2011-11-04]

## Beschreibung

Die vorliegende Erfindung betrifft ein Mittel zum Feststellen der Schwangerschaftswoche.

Zum Nachweis von Analyten, wie beispielsweise Schwangerschaftshormonen, in flüssigen Proben wie Urin und Speichel werden seit geraumer Zeit mit Reagenzien imprägnierte Teststreifen eingesetzt. Dabei wird üblicherweise die flüssige Probe mit einem Teststreifen in Kontakt gebracht, welcher einen porösen und saugfähigen Träger umfasst. Dieser Träger weist in einem ersten Bereich einen an den Analyten bindenden ersten spezifischen Bindungspartner auf, der im feuchten Zustand des Trägers in diesem frei beweglich ist. In einem weiteren Bereich befindet sich üblicherweise ein auf dem Träger immobilisierter, an den gleichen Analyten bindender zweiter spezifischer Bindungspartner. Der erste mobilisierbare Bindungspartner ist bei derartigen Vorrichtungen seinerseits an eine signalgebende Komponente gebunden (d.h. mit einem Farbstoff markiert). Bringt man nun den Teststreifen mit einer flüssigen Probe in Kontakt, so wird die Flüssigkeit in den ersten Bereich des Trägers eingesaugt, wo der Analyt mit dem ersten spezifischen Bindungspartner, an welches die signalgebende Komponente gebunden ist, reagiert. Der dabei gebildete Komplex wandert mit Hilfe der in dem Träger weitergesaugten Flüssigkeitsprobe zu dem immobilisierten zweiten Bindungsreagenz, mit welchem der Analyt reagiert, so dass in dem zweiten Bereich des Trägers, der als Nachweiszone dient, die dort immobilisierte signalgebende Komponente festgestellt werden kann.

In den meisten Fällen werden als spezifische Bindungspartner Analyt-spezifische Antikörper oder funktionelle Fragmente davon, die in der Lage sind, mit dem nachzuweisenden Analyten eine Bindung einzugehen. Bei den signalgebenden Komponenten kann es sich um Radioisotope, vorzugsweise aber um Farbstoffe, gefärbte Partikel oder Partikel mit Eigenfärbung handeln. Je nach der verwendeten signalgebenden Komponente ist in der Nachweiszone der Analyt visuell oder durch geeignete Geräte zu bestimmen, wobei für Teststreifen, die vom Endbenützer eingesetzt werden, die visuelle Darstellung am vorteilhaftesten ist.

Teststreifen, insbesondere häusliche Teststreifen, sind die am häufigsten angewandten Mittel zum Schwangerschaftsnachweis. Üblicherweise beruht der Schwangerschaftsnachweis mittels Teststreifen auf dem Nachweis des Schwangerschaftshormons hCG (= humanes Choriogonadotropin) im Urin. Dieses Hormon wird aus den Zellen der frühen Form des Mutterkuchens - so genannte Synzytiotrophoblasten - gebildet. Das Hormon bewirkt die weitere Ausschüttung von Östrogenen und Gestagenen aus dem Gelbkörper im Eierstock, ähnlich dem luteinisierenden Hormon (LH) aus der Hirnanhangsdrüse. Liegt keine Schwangerschaft vor, bildet sich der Gelbkörper zurück, damit die Gebärmutterschleimhaut abgebaut wird und in der Folge eine Periodenblutung stattfinden kann.

Die Konzentration von hCG im Urin verdoppelt sich am Anfang der Schwangerschaft alle 2,5 Tage, bis sie sich ab der 8. bis 10. Schwangerschaftswoche um 45.000 IE/l einpendelt. Kein Teststreifen im Stand der Technik, insbesondere solche die für den häuslichen Gebrauch bestimmt sind, ermöglicht es jedoch die Schwangerschaftswoche am Beginn der Schwangerschaft (ab der 8. bis zur 10. Schwangerschaftswoche) festzustellen.

Es ist Aufgabe der vorliegenden Erfindung Mittel zur Verfügung zu stellen, welche es ermöglichen, nicht nur eine Schwangerschaft, sondern insbesondere die Schwangerschaftswoche festzustellen.

GB 2 460 660 beschreibt einen Testapparat, mit dem die Zeit der Befruchtung bestimmt werden kann. Dabei wird in einer flüssigen Probe die Menge an hCG gemessen und in ein Signal umgewandelt. Dieses Signal wird mit einem Schwellenwert verglichen, wobei der Schwellenwert der Zeit der Befruchtung entspricht.

Die US 2010/129935 beschreibt einen Kit eines Urin-hCG-Tests für das Voranschreiten einer Schwangerschaft.

Die WO 2006/100415 offenbart einen Lateralflussimmunassay, mit dem hCG aus Urin detektiert wird.

Die EP 0 225 054 offenbart einen zweiseitigen Sandwichassay mit monoklonalen Antikörpern in der Reagenz- und Indikatorzone. Der Test kann als Schwangerschaftsindikator benutzt werden, der die Farbe verändert, wenn mehr als 200mIU/ml, d.h. 250, 300 und 500 mIU/ml hCG im Urin nachzuweisen sind.

Diese Aufgabe wird durch einen Kit zum Feststellen der Schwangerschaftswoche gelöst, welcher umfasst:
- eine Testvorrichtung mit einem porösen saugfähigen Träger umfassend einen ersten Bereich, der mit einem an humanem Choriongonadotropin (hCG) bindenden ersten spezifischen Bindungspartner, welcher in feuchtem Zustand des Trägers im Träger frei beweglich ist, imprägniert ist und umfassend einen zweiten Bereich als Nachweiszone, der ein auf dem Träger immobilisierten, an hCG bindenden zweiten spezifischen Bindungspartner aufweist, wobei der erste spezifische Bindungspartner mit einem Farbstoff markiert oder an einen Farbstoff, welcher stromabwärts vom ersten Bereich am Träger in feuchtem Zustand mobilisierbar imprägniert ist, markierbar ist, wobei der am Träger immobilisierte zweite spezifische Bindungspartner mindestens 2500 mIU hCG/mL Probe binden kann, und
- eine Farbskala zum visuellen Vergleich der Farbintensität, wobei den einzelnen Farbintensitäten der Skala eine bestimmte hCG Konzentration der zu untersuchenden Probe bzw. Schwangerschaftswoche wie folgt zugeordnet ist:

| **hCG-Konzentration [mIU/mL]** | **Schwangerschaftswoche** |
|---|---|
| < 100 | 1-2 |
| 100-2500 | 2-3 |
| > 2500 | 3 und mehr |

Der erfindungsgemäße Kit ermöglicht es in einfacher Weise neben einer vorhandenen Schwangerschaft auch die Schwangerschaftswoche zu bestimmen. Mit der darin vorgesehenen Testvorrichtung kann hCG, welches während einer Schwangerschaft in der menschlichen Plazenta gebildet wird und für die Erhaltung der Schwangerschaft verantwortlich ist, bestimmt werden. Je nach Menge des in der Probe enthaltenen hCGs färbt sich der zweite Bereich der Vorrichtung, die Nachweiszone, unterschiedlich intensiv an. Die Intensität der Färbung ist einerseits von der tatsächlichen Konzentration von hCG in der Probe und andererseits von der Inkubationszeit der Testvorrichtung abhängig. Um schlussendlich die Konzentration an hCG in der Probe und die Schwangerschaftswoche, welche mit der Konzentration von hCG zumindest in den ersten 8 bis 10 Wochen direkt korreliert, zu bestimmen, wird die Farbe bzw. Farbintensität in der Nachweiszone mit der Farbe bzw. Farbintensität einer Farbskala verglichen. In dieser Farbskala sind die Farben bzw. Intensitäten, die an der Testvorrichtung erscheinen können, den hCG Konzentrationen bzw. den Schwangerschaftswochen zugeordnet. Die Farbskala wird dem jeweiligen verwendeten Farbstoff angepasst. Die hCG-Konzentrationen korrelieren mit der Schwangerschaftswoche wie folgt:

| **HCG-Konzentration [mIU/mL]** | **Schwangerschaftswoche** |
|---|---|
| < 100 | 1-2 |
| 100-2500 | 2-3 |
| > 2500 | 3 und mehr |

Als Farbstoff kann in der erfindungsgemäßen Testvorrichtung jeglicher Farbstoff verwendet werden, der geeignet ist an einen hCG-spezifischen Bindungspartner zu binden. Zusätzlich ist es von Vorteil, wenn der Farbstoff ohne Zuhilfenahme zusätzlicher technischer Hilfsmittel für den Benutzer mit freiem Auge sichtbar ist. Derartige Farbstoffe werden regelmäßig in, z.B., Teststreifen eingesetzt und sind somit dem Fachmann hinreichend bekannt. Alternativ dazu ist es auch möglich, stromabwärts des ersten Bereichs in Richtung des zweiten Bereichs bzw. der Nachweiszone einen Farbstoff vorzusehen, welcher in feuchtem Zustand der Testvorrichtung mobilisiert werden kann, der in der Lage ist an den ersten hCG spezifischen Bindungspartner zu binden. Die Markierung des hCG spezifischen Bindungspartners kann, wie oben diskutiert, direkt zu einem sichtbaren Signal führen. Der spezifische Bindungspartner kann jedoch auch indirekt detektierbar sein, indem dieser z.B. mit einem Enzym, Biotin- oder Avidin, die in einer oder mehreren Reaktionen teilnehmen, markiert ist, um eine detektierbare Substanz zu erzeugen. Besonders bevorzugt wird die Enzymmarkierung eingesetzt, insbesondere mit Peroxidase, Glucoseoxidase, β-Galactosidase oder alkalischer Phosphatase. Im Fall einer indirekten Markierung des hCG spezifischen Bindungspartners ist oder sind die entsprechenden Reaktionspartner stromabwärts vom ersten Bereich und vor dem zweiten Bereich (Nachweiszone) am Träger vorgesehen.

Im Sinne dieser Erfindung beschreibt der Begriff "stromabwärts" jene Seite bzw. Richtung in der erfindungsgemäßen Vorrichtung, welche von der Probenauftragszone wegführt. D.h. "stromabwärts" gibt die Flussrichtung der Flüssigkeit (basierend auf der Kapillarwirkung des porösen Trägers) von der Probenauftragszone zur Detektionszone und zu einem etwaigen Kontrollbereich innerhalb der Testvorrichtung an.

Der prinzipielle Aufbau der Testvorrichtung (wie z.B. Träger) gemäß der vorliegenden Erfindung entspricht im Wesentlichen jenen, welche bereits im Stand der Technik, insbesondere in der EP 0 383 619, EP 0 362 809, GB 1 589 234, EP 0 225 054 oder der WO 88/08534, ausführlich beschrieben sind.

Gemäß einer bevorzugten Ausführungsform der vorliegenden Erfindung ist der an den Analyten bindende erste und zweite spezifische Bindungspartner ein Antikörper oder ein funktionelles Fragment davon.

Analyt-bindende Antikörper können mit Hilfe bekannter Verfahren hergestellt und auf einen geeigneten porösen Träger immobilisiert werden.

Der erfindungsgemäße Träger weist in einem zweiten Bereich, der Nachweiszone, immobilisierte Bindungspartner auf, welche ebenfalls an hCG binden können. Durch das Vorsehen von immobiliserten Bindungspartnern ist es möglich, die mit einem Farbstoff direkt oder indirekt markierten Komplexe aus hCG und ersten hCG spezifischen Bindungspartner an einer Stelle des Trägers zu binden. Vorzugsweise wird die Menge des zweiten Bindungspartners so gewählt, dass in diesem Bereich eine bestimmte Menge an hCG gebunden werden kann.

Gemäß einer bevorzugten Ausführungsform der vorliegenden Erfindung kann der am Träger immobilisierte zweite spezifische Bindungspartner mindestens 3000 mIU hCG/mL Probe, vorzugsweise mindestens 4000 mIU hCG/mL Probe, binden, wobei vorzugsweise ca. 0,5 bis 1 ml Probe, insbesondere Urin, aufgetragen wird.

Um die Analyten in der Probe visuell zu markieren, dass diese in der Nachweiszone sichtbar werden, sind die ersten Bindungspartner mit einem signalgebenden Stoff markiert, der vorzugsweise ein Farbstoff, ausgewählt aus der Gruppe bestehend aus kolloidalem Gold, gefärbten Latex-Partikeln, wasserlöslichen Farbstoffen, wie z.B. Rhodamin, radioaktive oder fluoreszierende Farbstoffe, ist.

Besonders bevorzugt werden erfindungsgemäß Farbstoffe eingesetzt, welche vorzugsweise ausgewählt sind aus der Gruppe bestehend aus optisch sichtbaren Farbstoffen, umfassend lösliche optisch sichtbare Farbstoffe, wie Pigmente, Küpenfarbstoffe, schwefelhaltige Farbstoffe, Beizfarbstoffe, Leukoformen von Küpenfarbstoffen und Farbstoffarten, wie Fluorescein, Rhodamin und Derivate davon (wie Sulphorhodamin, Rhodaminhydrid und Rhodaminhydrazid), wie auch Oxazinfarbstoffe, Cyaninfarbstoffe und Azolfarbstoffe. Spezielle Beispiele von geeigneten Farbstoffen sind beispielsweise Texas-Rot-Hydrazin, Congo-Rot, Trypan-Blau, Lissamin-Blau, Remazol-Schwarz, Remazol-Rot, Rhodamine-B-Isothiocyanat, Cy5-Osu-monofunktionell-reaktiver Farbstoff, Reaktiv-Orange 16, Uniblue A usw.

Stromabwärts zum zweiten Bereich bzw. zur Nachweiszone ist vorzugsweise ein Kontrollbereich vorgesehen, in dem Bindungspartner immobilisiert sind, die spezifisch an den mobilisierbaren hCG bindenden ersten spezifischen Bindungspartner binden. Dieser Kontrollbereich dient dazu, um dem Benutzer zu zeigen, dass die Testvorrichtung funktionstüchtig ist, und dass die Probe bereits die Nachweiszone passiert hat. Da der erste Bindungspartner entweder direkt oder indirekt markiert ist, sobald er zum Kontrollbereich gelangt, sieht der Benutzer der erfindungsgemäßen Vorrichtung sofort, dass die Probe die Nachweiszone bereits passiert hat und der Test dadurch im Wesentlichen abgeschlossen ist.

Ein noch weiterer Aspekt der vorliegenden Erfindung betrifft ein Verfahren zum Feststellen der Schwangerschaftswoche, umfassend die Schritte:
- Bereitstellen einer Probe, vorzugsweise einer Urinprobe, einer schwangeren Patientin,
- Auftragen der Probe auf einen Probenaufgabebereich einer Testvorrichtung gemäß einem der Ansprüche 1 bis 4,
- Inkubieren der Testvorrichtung über einen Zeitraum von mindestens 30 Sekunden,
- visuelles Vergleichen der Farbintensität in der Nachweiszone der Testvorrichtung mit einer Farbskala, wobei den einzelnen Farbintensitäten der Skala eine bestimmte hCG Konzentration bzw. Schwangerschaftswoche wie folgt zugeordnet ist:

| **hCG-Konzentration [mIU/mL]** | **Schwangerschaftswoche** |
|---|---|
| < 100 | 1-2 |
| 100-2500 | 2-3 |
| > 2500 | 3 und mehr |

und
- Bestimmen der Schwangerschaftswoche durch Zuordnen der Farbintensität in der Nachweiszone zu den Farbintensitäten der Skala.

Mit dem erfindungsgemäßen Verfahren kann der Benutzer die Schwangerschaftswoche rasch und einfach bestimmen, indem er nach Abschluss des Tests die etwaig auftretende Farbe in der Nachweiszone mit einer Farbskala optisch vergleicht.

Gemäß einer bevorzugten Ausführungsform der vorliegenden Erfindung wird die Testvorrichtung über einen Zeitraum von mindestens 1 Minute, vorzugsweise mindestens 3 Minuten, vorzugsweise mindestens 4 Minuten, insbesondere von 5 Minuten, vorzugsweise bei Raumtemperatur inkubiert.

In einer alternativen Ausführungsform der vorliegenden Erfindung wird die Testvorrichtung so lange inkubiert bis die Nachweiszone eine definierte Farbstärke erreicht. Diese Farbe kann wiederum einer Farbskala entnommen werden. Der Zeitraum vom Probenauftrag zum Erreichen der vordefinierten Farbstärke zeigt an, welche Konzentration an hCG in der Probe vorhanden ist. Je schneller eine bestimmte Farbstärke erreicht wird, desto mehr hCG befindet sich in der Probe.

Die erfindungsgemäße Testvorrichtung ist vorzugsweise ein Teststreifen.

Die erfindungsgemäße Testvorrichtung kann Teil eines Testgeräts sein, welches dadurch gekennzeichnet ist, dass der Behälter aus zwei länglichen, insbesondere schalenförmigen Behälterhälften gebildet ist, die an ihren einander zugewandten Längsrändern miteinander verbunden sind. Vorzugsweise besteht dabei zumindest eine Behälterhälfte zur Gänze aus transparentem Material, um die Herstellung besonders einfach zu gestalten. Die zwei Behälterhälften sind vorzugsweise im Wesentlichen identisch ausgebildet, wobei beim Zusammensetzen der Teststreifen in die eine Behälterhälfte eingelegt wird, und die beiden Behälterhälften fest, etwa durch Verkleben oder Verschweißen, z.B. Ultraschallschweißen, miteinander verschlossen werden. Bevorzugt wird jedoch eine Steck-, Rast- oder Schnappverbindung, da dadurch ein zusätzlicher Arbeitsschritt beim Zusammenbau vermieden wird. Ein Öffnen des Behälters ist danach nur noch mit einem gewissen Kraftaufwand oder gar nicht mehr möglich, so dass der Teststreifen selbst bei gröberer Handhabung fest in den zwei Behälterhälften verschlossen und vor jeglicher Verschmutzung geschützt ist. Es kann jeder herkömmliche Teststreifen eingesetzt sein, wichtig ist nur, dass die zu testende Flüssigkeit, zumeist Harn, durch die zumindest eine Einlassöffnung in das Behälterinnere zur Testzone des Teststreifens, bzw. im Fall von überschüssigem Harn, durch die Auslassöffnung wieder nach außen gelangen kann. Das Testgerät kann entweder in einen Behälter mit gesammeltem Harn getaucht oder in einen Harnstrahl (in den Mittelstrahl) gehalten werden. Zur einfacheren Herstellung ist bevorzugt, wie erwähnt, die eine Behälterhälfte zur Gänze durchsichtig auszubilden, wobei die zweite Behälterhälfte ohne weiteres undurchsichtig ausgebildet werden kann. Möglich wäre es aber auch, nur einen fensterartigen durchsichtigen Testzonen-Bereich vorzusehen, oder aber auch beide Behälterhälften gleich transparent auszubilden. An sich könnte auch die Sichtzone durch eine einfache Ausnehmung, ein Fenster bzw. eine Öffnung gebildet sein.

Denkbar ist es im Übrigen auch, nur eine Behälterhälfte schalenförmig auszubilden und die andere Behälterhälfte als flachen Bodenteil vorzusehen.

Im Fall einer Steck-, Rast- oder Schnappverbindung besteht eine Möglichkeit darin, in der einen Behälterhälfte Steckzapfen und in der anderen Behälterhälfte dazu entsprechende Löcher vorzusehen, wobei die Steckzapfen zum Verschließen der Behälterhälften in die Löcher eingedrückt werden. Dies ist ein einfacher und dennoch äußerst effektiver Verschluss. Günstig ist es hierbei, wenn solche Steckzapfen/Loch-Anordnungen allseitig um den Innenraum herum angebracht sind, so dass auf jeder Seite des Behälters ein derartiger Verschluss vorhanden ist.

Vorteilhafterweise sind beide Behälterhälften mit einer Einlassöffnung in Abstand vom Testzonen-Bereich ausgebildet. Dadurch wird eine beidseitige Benutzung ermöglicht. Möglich ist jeweils eine runde, eckige oder bevorzugt eine Querschlitz-förmige Einlassöffnung pro Behälterhälfte, selbstverständlich können auch zwei oder mehr Einlassöffnungen pro Behälterhälfte vorgesehen sein. Günstig wirkt sich dabei eine von der (den) Einlassöffnung(en) aus zum Inneren des Testgeräts hin abgeschrägte Fläche für ein ruhiges, laminares Einfließen des Harns in das Innere aus. Aus Gründen der einfacheren Herstellung liegen die Einlassöffnungen der beiden Behälterhälften einander direkt gegenüber. Die Einlassöffnungen sind zweckmäßig nicht direkt im Bereich der Testzone ausgebildet, so dass der Harn nicht direkt auf die Testzone auftreffen kann, sondern - wie bekannt - mittels Kapillarwirkung "gesteuert" zur Testzone gelangt.

Für einen sicheren Eintritt des Harns in den Behälter ist es von Vorteil, wenn die Einlassöffnung(en) im Anschluss an eine an der Außenseite der Behälterhälfte gebildete Zulaufrinne vorgesehen ist (sind). Diese Zulaufrinne kann z.B. durch eine Vertiefung der Behälterhälfte-Oberfläche gebildet sein, wobei es besonders günstig ist, wenn sich die Vertiefung zur Einlassöffnung hin vertieft. Die Länge der Zulaufrinne ist variabel, in der Regel wird sie etwa 0,1 bis 2 cm betragen. Durch die Zulaufrinne wird der Harn zu der (den) Einlassöffnung(en) geführt. Selbst bei geringen Harnmengen bzw. bei zähflüssigerem, trübem, lipämischem Harn ist damit ein Test mit sicherem Ergebnis möglich. Vorzugsweise ist, um eine dünnwandige Ausbildung der Behälterhälften zu ermöglichen, die Zulaufrinne durch zwei von der Wand der Behälterhälfte hochstehende Stege gebildet. Die Stege sind zweckmäßig einteilig mit der Behälterhälfte ausgebildet. Durch die Stege ist eine Vertiefung der Behälterwand nicht oder nur in geringem Ausmaß notwendig. Eine hinsichtlich der Harnführung besonders vorteilhafte Form der Zulaufrinne ist dadurch gegeben, dass die Stege einen sich in Richtung zu einer vorzugsweise Querschlitz-förmigen Einlassöffnung hin verkleinernden Abstand voneinander haben.

Vorzugsweise ist die zumindest eine Auslassöffnung an der einen Stirnseite zumindest einer Behälterhälfte vorgesehen. Damit ist sichergestellt, dass der Harn zunächst über einen Teil des Teststreifens strömt und erst dann stirnseitig austritt.

Eine weitere baulich und funktionell günstige Form ist dadurch gegeben, dass die zumindest eine Auslassöffnung durch zwei zueinander ausgerichtete rinnenförmige Ausnehmungen in den einander zugewandten Rändern der Behälterhälften gebildet ist. Dadurch ist gewährleistet, dass die Auslassöffnung nicht einseitig, sondern symmetrisch in der Höhenmitte des Behälters, angebracht ist, so dass nicht erst ein Kippen des Behälters in die eine Richtung notwendig ist, damit ein Harnüberschuss aus dem Behälter austritt. Möglich ist eine einzige Auslassöffung, bevorzugt sind aber (zumindest) zwei Auslassöffnungen an der Behälter-Stirnseite.

Ein zusätzlicher Vorteil bei der Benützung des Testgeräts ergibt sich, wenn jede Behälterhälfte an einem stirnseitigen Ende mit einem verbreiterten Griffteil ausgebildet ist. Somit ist an dem einen Behälterende ein benutzerfreundlicher Griff ausgebildet, wodurch eine bessere Handhabung und automatisch ein richtiges Halten des insgesamt löffelartig ausgebildeten Testgeräts während der Benützung gesichert ist. Dabei ist (sind) die Auslassöffnung(en) an der dem Griff gegenüberliegenden Stirnseite vorgesehen.

Eine besonders günstige Form ist dadurch gekennzeichnet, dass beide Behälterhälften mit einem Quersteg geformt sind, der eine geringere Höhe aufweist, verglichen mit der Tiefe der jeweiligen Innenraum-Hälfte, und der einen Harn-Aufnahmebereich, in dem sich die Einlassöffnung befindet, vom Testzonen-Bereich trennt. Bevorzugt ist (sind) auch die Auslassöffnung(en) in dem Harn-Aufnahmebereich ausgebildet. Dadurch wird ein freies Fließen des Harns durch das gesamte Behälterinnere verhindert. Die Querstege lassen lediglich einen dünnen Spalt frei, in dem der Teststreifen eingeklemmt wird, so dass eine Art Abdichtung des Testzonen-Bereichs erhalten wird. Möglich ist es natürlich auch, dass Zusatzteile mit dem Teststreifen zwischen den zwei Behälterhälften, eventuell den Querstegen, eingeklemmt werden, wie etwa ein eigenes Dichtungsstück.

Vorzugsweise ist im Harn-Aufnahmebereich ein Speicherkörper aufgenommen, von dem aus der Harn mittels Kapillarwirkung zur Testzone gelangt. Dieser Speicherkörper, der günstigerweise das gesamte Volumen des Harn-Aufnahmeraums ausfüllt, ist aus einem Stoff gebildet, der Flüssigkeiten aufnimmt, etwa ein Faser- oder Schaumstoffkörper. Eine exakt gesteuerte, ausreichende Harnleitung zur Testzone wird dadurch erreicht, dass der Speicherkörper zusammen mit dem Teststreifen zwischen den Querstegen eingeklemmt wird, so dass Harn vom Aufnahmebereich bzw. vom dort vorhandenen Speicherkörper über den Teststreifen zwischen den Querstegen zur Testzone gelangt.

Von Vorteil ist es auch, wenn die beiden Behälterhälften aus Kunststoff, wie Polystyrol, gespritzt sind. Dieses Material sichert einen guten Schutz des Teststreifens, ist stabil, und die Herstellung durch Spritzen ist besonders einfach und kostengünstig in der Serienproduktion.

Die Erfindung wird nachstehend anhand eines in der Zeichnung dargestellten bevorzugten Ausführungsbeispiels, auf die sie jedoch nicht beschränkt sein soll, noch weiter erläutert.

Im Einzelnen zeigen Fig. 1 eine Draufsicht auf ein Testgerät; Fig. 2 eine Seitenansicht dieses Testgeräts; Fig. 3 eine Draufsicht auf das Innere einer Behälterhälfte; Fig. 4 einen Querschnitt dieses Testgeräts und Fig. 5 eine erfindungsgemäße Testvorrichtung in Form eines Teststreifens.

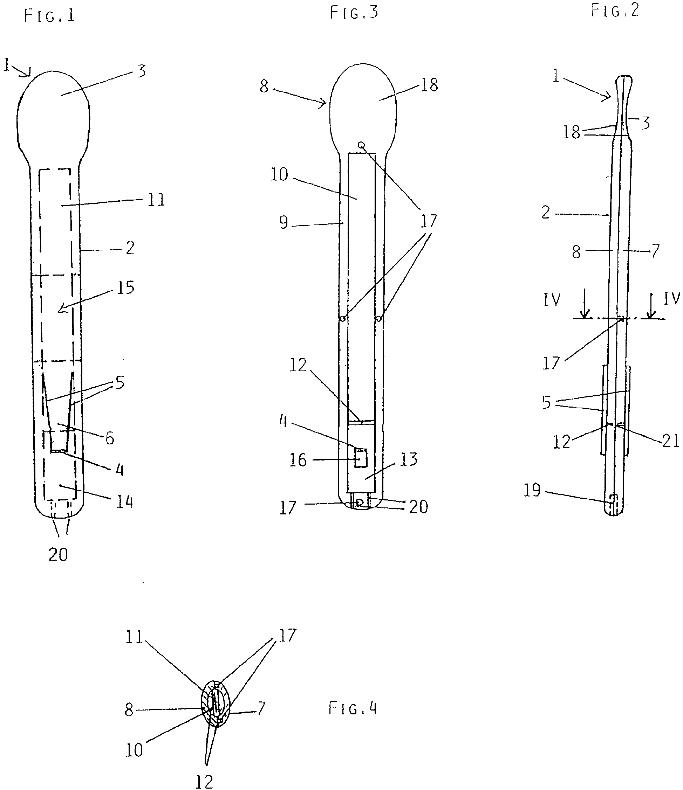

Die Figuren 1 und 2 zeigen in einer Draufsicht bzw. Seitenansicht ein insgesamt löffelartiges Testgerät 1 mit einem Behälter 2 mit einem Griff 3 an einem stirnseitigen Ende und einer Querschlitz-förmigen Einlassöffnung 4 nahe dem anderen stirnseitigen Ende des allgemein rohrförmigen Behälters 2. Weiters sind zwei hochstehende Stege 5 ersichtlich, die einen sich in Richtung der Einlassöffnung 4 hin verkleinernden Abstand voneinander haben. Die Stege 5 bilden somit eine Zulaufrinne 6 für den zur Einlass-Öffnung 4 fließenden Harn.

Der Behälter 2 besteht aus zwei im Wesentlichen gleichen, schalenförmigen Behälterhälften 7, 8, s. auch Fig. 4. In Fig. 3 ist die eine untere Behälterhälfte 8 des Testgeräts 1 von innen dargestellt, wobei ein von einem Rand 9 umgebener Innenraum 10, in dem ein Teststreifen (11 in Fig. 1 und 4) mit eingelegt wird. Durch Querstege 12 ist der Innenraum 10 hinsichtlich eines Harn-Aufnahmebereichs 13, in dem ein Speicherkörper 14 (Fig. 1) eingelegt wird, und eines Testzonen-Bereichs 15 getrennt. Dieser Testzonen-Bereich 15 kann durch Beschriftungen am Teststreifen 11 gekennzeichnet oder durch über dem Teststreifen 11 angebrachte färbige Abdeckungen (nicht gezeigt) begrenzt sein. Im Harn-Aufnahmebereich 13 ist die Querschlitz-förmige Einlassöffung 4 ersichtlich, wobei daran eine abgeschrägte Fläche 16 anschließt, so dass das Einfließen des Harns in den Harn-Aufnahmebereich 13 gleichmäßig erfolgt. Am Rand 9 des Testgeräts l sind vier Steckzapfen 17 ersichtlich, die in die vier korrespondierenden Löcher der zweiten Behälterhälfte 7 steckbar sind, um eine feste Verbindung der beiden Behälterhälften 7, 8 zu gewährleisten. Für eine einfache Herstellung kann es auch günstig sein, wenn jede Behälterhälfte 7 (bzw. 8) sowohl Löcher als auch Steckzapfen 17 aufweist, wobei gleich viele Löcher wie Steckzapfen 17 vorhanden sind, die symmetrisch bezüglich der Längsachse des Behälters 2 angeordnet sind. Auf diese Weise muss lediglich eine Behälterhälfte 7 (bzw. 8) hergestellt werden: Zwei gleiche Behälterhälften 7 (bzw. 8) können aufeinander gelegt werden, wobei die Steckzapfen 17 in die entsprechenden Löcher eingesteckt werden.

Jede Behälterhälfte 7, 8 ist einstückig aus Kunststoff, z.B. Polystyrol bzw. ABS, gespritzt und dabei mit einem Griffteil 18 geformt, wobei die beiden Griffteile 18 den Griff 3 ergeben. An dem Griff 3 des Testgeräts l gegenüberliegenden stirnseitigen Ende sind zwei Austrittsöffnungen 19 vorgesehen, die je durch rinnenförmige Ausnehmungen oder Vertiefungen 20 im Rand 9 gebildet sind, die, wenn die zwei Behälterhälften 7, 8 aufeinander gelegt werden, die zwei Auslassöffnungen 19 bilden. Dadurch wird ein Harnfluss (bei einem Überschuss an Harn) in eine Richtung, nämlich von der Einlassöffung 4 durch den Harn-Aufnahmebereich 13 bis zu den Auslassöffungen 19 gewährleistet.

Fig. 2 zeigt in der Seitenansicht des Testgeräts l die Wölbung des Griffs 3. Weiters sind die zwei Querstege 12 gezeigt, wobei ersichtlich ist, dass sie eine vergleichsweise geringere Höhe im Innenraum 10 haben und somit einen Spalt 21 zwischen ihnen belassen, in den lediglich der Teststreifen 11 sowie eventuelle andere Streifen, z.B. mit Informationen, oder der zusammengepresste Speicherkörper 14, passen.

Die Zulaufrinnen 6 zu den Einlassöffnungen 4 sind beide durch hochstehende Stege 5 gebildet.

Fig. 4 stellt das Testgerät l im Querschnitt gemäß der Linie IV-IV in Fig. 2 dar, wobei der Innenraum 10 durch die zwei Behälterhälften 7, 8 definiert wird, die durch die Steckverbindung mit den Steckzapfen 17 zusammengehalten werden. In dem Innenraum 10 ist ein Teststreifen 11 gezeigt. Weiters sind die Querstege 12 angedeutet.

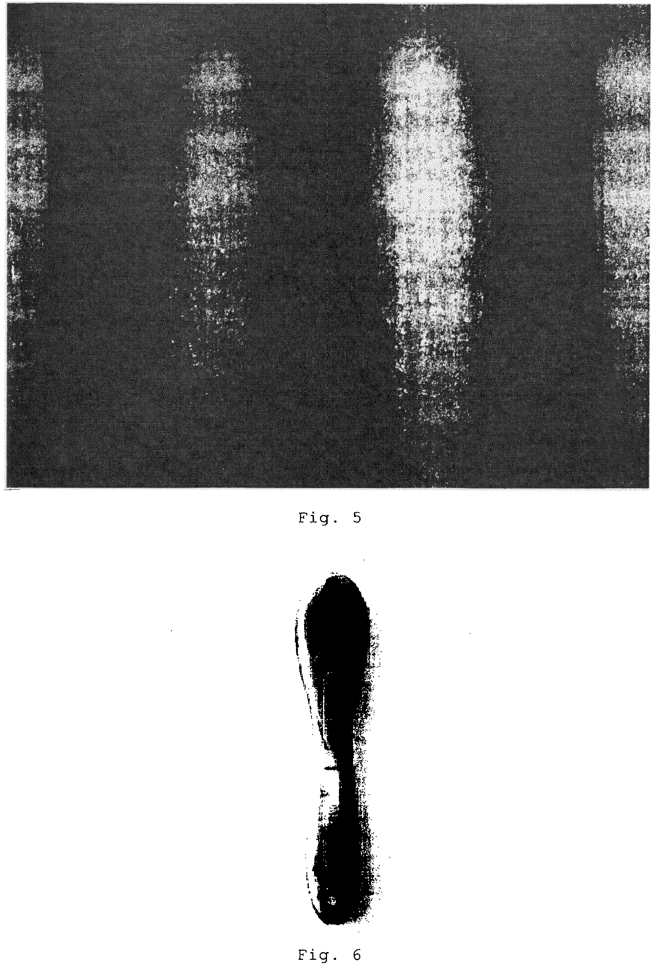

Fig. 5 und Fig. 6 zeigen drei erfindungsgemäße Testgeräte bzw. ein Testgerät mit einer darin eingebrachten Testvorrichtung. Die abgebildeten Testvorrichtungen weisen eine Nachweiszone (Bereich) mit einer Menge an immobilisierten Anti-hCG-Antikörper von 0,1 mMcroliter/mm mit der Konzentration von 4 mg/ml auf, wobei die Konzentration auch entsprechend variiert werden kann. In der linken Testvorrichtung wurde eine Probe mit 25 mUI/mL hCG, in der Mitte mit 529 mUI/mL hCG und in der rechten Vorrichtung mit 2022 mUI/mL hCG aufgetragen. Aus dieser Figur ist ersichtlich, dass die Nachweiszone je nach hCG Konzentration unterschiedlich intensiv gefärbt ist. Ein Vergleich dieser Färbungen mit einer Farbskala, lässt auf die hCG Konzentration rückschließen. Der farbige Bereich stromabwärts der Nachweiszone ist die Kontrollzone, in der gefärbtes nicht an hCG gebundener und markierter hCG Bindungspartner immobilisiert wird.

## Patentansprüche

1. Kit zum Feststellen der Schwangerschaftswoche, umfassend:
- eine Testvorrichtung mit einem porösen saugfähigen Träger umfassend einen ersten Bereich, der mit einem an humanem Choriongonadotropin (hCG) bindenden ersten spezifischen Bindungspartner, welcher in feuchtem Zustand des Trägers im Träger frei beweglich ist, imprägniert ist und umfassend einen zweiten Bereich als Nachweiszone, der ein auf dem Träger immobilisierten, an hCG bindenden zweiten spezifischen Bindungspartner aufweist, wobei der erste spezifische Bindungspartner mit einem Farbstoff markiert oder an einen Farbstoff, welcher stromabwärts vom ersten Bereich am Träger in feuchtem Zustand mobilisierbar imprägniert ist, markierbar ist, wobei der am Träger immobilisierte zweite spezifische Bindungspartner mindestens 2500 mIU hCG/mL Probe binden kann, und
- eine Farbskala zum visuellen Vergleich der Farbintensität, wobei den einzelnen Farbintensitäten der Skala eine bestimmte hCG Konzentration der zu untersuchenden Probe bzw. Schwangerschaftswoche wie folgt zugeordnet ist:
| **hCS-Konzentration [mIU/mL]** | **Schwangerschaftswoche** |
|---|---|
| < 100 | 1-2 |
| 100-2500 | 2-3 |
| > 2500 | 3 und mehr |

2. Kit gemäß Anspruch 1, **dadurch gekennzeichnet, dass** der an hCG bindende erste und zweite spezifische Bindungspartner ein Antikörper oder ein funktionelles Fragment davon ist.

3. Kit gemäß Anspruch 1 oder 2, **dadurch gekennzeichnet, dass** der am Träger immobilisierte zweite spezifische Bindungspartner mindestens 3000 mIU hCG/mL Probe, vorzugsweise 4000 mIU hCG/mL Probe, binden kann.

4. Kit gemäß einem der Ansprüche 1 bis 3, **dadurch gekennzeichnet, dass** stromabwärts zum zweiten Bereich bzw. zur Nachweiszone ein Kontrollbereich vorgesehen ist, in dem Bindungspartner immobilisiert sind, die spezifisch an den hCG bindenden ersten spezifischen Bindungspartner binden.

5. Verfahren zum Feststellen der Schwangerschaftswoche, umfassend die Schritte:
- Bereitstellen einer Probe, vorzugsweise einer Urinprobe, einer schwangeren Patientin,
- Auftragen der Probe auf einen Probenaufgabebereich einer Testvorrichtung gemäß einem der Ansprüche 1 bis 4,
- Inkubieren der Testvorrichtung über einen Zeitraum von mindestens 30 Sekunden,
- visuelles Vergleichen der Farbintensität in der Nachweiszone der Testvorrichtung mit einer Farbskala, wobei den einzelnen Farbintensitäten der Skala eine bestimmte hCG Konzentration bzw. Schwangerschaftswoche wie folgt zugeordnet ist:
| **hCG-Konzentration [mIU/mL]** | **Schwangerschaftswoche** |
|---|---|
| < 100 | 1-2 |
| 100-2500 | 2-3 |
| > 2500 | 3 und mehr |
und
- Bestimmen der Schwangerschaftswoche durch Zuordnen der Farbintensität in der Nachweiszone zu den Farbintensitäten der Skala.

6. Verfahren nach Anspruch 5, **dadurch gekennzeichnet, dass** die Testvorrichtungen über einen Zeitraum von mindestens 1 Minute, vorzugsweise mindestens 3 Minuten, insbesondere von 5 Minuten, vorzugsweise bei Raumtemperatur inkubiert wird.

## Claims

1. Kit for determining the week of pregnancy, comprising:
- a testing device having a porous absorbent support comprising a first region, which is impregnated with a first specific binding partner which binds to human chorionic gonadotropin (hCG) and which is freely movable in the support when the support is moist, and comprising a second region as a detection zone, which has a second specific binding partner which is immobilized on the support and which binds to hCG, wherein the first specific binding partner is labeled with a dye or can be labeled with a dye that is impregnated on the support downstream of the first area such that the dye can be mobilized in a moist state, wherein the second specific binding partner which is immobilized on the support is capable of binding at least 2,500 mIU hCG/ml of a sample; and
- a color scale for visually comparing color intensities, wherein a specific hCG concentration of the sample to be analyzed/a week of pregnancy is assigned to the individual color intensities on the scale as follows:
| **hCG concentration [mIU/mL]** | **Week of pregnancy** |
|---|---|
| < 100 | 1 - 2 |
| 100 - 2,500 | 2 - 3 |
| > 2,500 | 3 and more |

2. Kit according to claim 1, **characterized in that** the first and second specific binding partner which binds to hCG is an antibody or a functional fragment thereof.

3. Kit according to claim 1 or 2, **characterized in that** the second specific binding partner which is immobilized on the support is capable of binding at least 3,000 mIU hCG/mL of a sample, preferably at least 4,000 mIU hCG/mL of a sample.

4. Kit according to any one of claims 1 to 3, **characterized in that** downstream of the second region/detection zone a control region is provided in which binding partners are immobilized which specifically bind to the hGC-binding first specific binding partner.

5. Method for determining the week of pregnancy, comprising the following steps:
- providing a sample, preferably a urine sample, obtained from a pregnant patient,
- applying said sample to a sample application region of a testing device according to any one of claims 1 to 4,
- incubating said testing device over a period of at least 30 seconds,
- visually comparing the color intensity in the detection zone of the testing device to a color scale, wherein a specific hCG concentration/week of pregnancy is associated to the individual color intensities on the scale as follows:
| **hCG concentration [mIU/mL]** | **Week of pregnancy** |
|---|---|
| < 100 | 1 - 2 |
| 100 - 2,500 | 2 - 3 |
| > 2,500 | 3 and more |
- determining the week of pregnancy by assigning the color intensity in the detection zone to the respective color intensity on the scale.

6. Method according to claim 5, **characterized in that** the testing device is incubated over a period of at least 1 minute, preferably at least 3 minutes, in particular of 5 minutes, preferably at room temperature.

## Revendications

1. Kit pour déterminer la semaine de grossesse, comportant :
- un dispositif de test comportant un support absorbant poreux comportant une première zone, qui est imprégnée d'un premier partenaire de liaison spécifique liant une choriongonadotropine (hCG) humaine, partenaire de liaison qui est librement déplaçable dans le support dans l'état humide du support, et comportant une seconde zone en tant que de zone de détection, qui présente un second partenaire de liaison spécifique liant hCG et immobilisé sur le support, dans lequel le premier partenaire de liaison spécifique est marqué avec un colorant ou peut être marqué dans un colorant, qui est imprégné en aval de la première zone dans le support étant mobilisable dans l'état humide, dans lequel le second partenaire de liaison spécifique immobilisé dans le support peut lier un échantillon d'au moins 2500 mIU hCG/mL, et
- une échelle de couleur pour une comparaison visuelle de l'intensité de couleur, dans laquelle une concentration en hCG déterminée de l'échantillon à examiner ou de la semaine de grossesse est associée à différentes intensités de couleur de l'échelle comme suit :
| Concentration en hCG (mIU/mL) | Semaine de grossesse |
|---|---|
| < 100 | 1-2 |
| 100-2500 | 2-3 |
| > 2500 | 3 et plus |

2. Kit selon la revendication 1, **caractérisé en ce que** le premier et le second partenaire de liaison spécifique liant une hCG est un anticorps ou un fragment fonctionnel de celui-ci.

3. Kit selon la revendication 1 ou 2, **caractérisé en ce que** le second partenaire de liaison spécifique immobilisé dans le support peut lier un échantillon d'au moins 3000 mIU hCG/mL, de préférence un échantillon de 4000 mIU hCG/mL.

4. Kit selon l'une des revendications 1 à 3, **caractérisé en ce que**, en aval de la seconde zone ou de la zone de détection est prévue une zone de contrôle, dans laquelle les partenaires de liaison sont immobilisés, qui lient spécifiquement un premier partenaire de liaison spécifique liant hCG.

5. Procédé pour déterminer la semaine de grossesse, comportant les étapes suivantes :
- préparer un échantillon, de préférence un échantillon d'urine, d'une patiente enceinte,
- appliquer l'échantillon sur une zone de dépôt d'échantillon d'un dispositif de test selon l'une des revendications 1 à 4,
- incuber le dispositif de test sur une durée d'environ 30 secondes,
- comparer visuellement l'intensité de couleur dans la zone de détection du dispositif de test à une échelle de couleur, dans laquelle une concentration en hCG déterminée ou une semaine de grossesse est associée à différentes intensités de couleur de l'échelle comme suit :
| Concentration en hCG (mIU/mL) | Semaine de grossesse |
|---|---|
| < 100 | 1-2 |
| 100-2500 | 2-3 |
| > 2500 | 3 et plus |
et
- déterminer la semaine de grossesse par association de l'intensité de couleur dans la zone de détection aux intensités de couleur de l'échelle.

6. Procédé selon la revendication 5, **caractérisé en ce que** les dispositifs de test sont incubés sur une durée d'au moins 1 minute, de préférence d'au moins 3 minutes, en particulier de 5 minutes, de préférence à température ambiante.
